(19) Europäisches Patentamt
European Patent Office
Office européen des brevets

(11) Numéro de publication: **0 246 149 B1**

(12) **FASCICULE DE BREVET EUROPEEN**

(45) Date de publication de fascicule du brevet: **30.12.92**

(51) Int. Cl.$^5$: **C12P 21/00**, C08B 37/00, A61K 39/39

(21) Numéro de dépôt: **87401057.2**

(22) Date de dépôt: **11.05.87**

(54) **Nouveaux immunomodulateurs obtenus par hémisynthèse à partir d'un polysaccharide bactérien isolé d'une souche mutante non capsulée de Klebsiella pneumoniae.**

(30) Priorité: **12.05.86 FR 8606765**

(43) Date de publication de la demande:
**19.11.87 Bulletin 87/47**

(45) Mention de la délivrance du brevet:
**30.12.92 Bulletin 92/53**

(84) Etats contractants désignés:
**AT BE CH DE ES FR GB GR IT LI LU NL SE**

(56) Documents cités:
**EP-A- 0 089 266**
**EP-A- 0 115 988**
**EP-A- 0 180 564**
**FR-A- 2 570 081**

(73) Titulaire: **PIERRE FABRE MEDICAMENT**
**45, Place Abel Gance**
**F-92100 Boulogne(FR)**

(72) Inventeur: **Dussourd D'Hinterland, Lucien**
**Domaine de Plombières Avenue de Lautrec**
**F-81100 Castres(FR)**
Inventeur: **Normier, Gérard**
**23, rue Francis Poullenc**
**F-81100 Castres(FR)**
Inventeur: **Pinel, Anne-Marie**
**22, rue des Capucins**
**F-81100 Castres(FR)**

(74) Mandataire: **Warcoin, Jacques et al**
**Cabinet Régimbeau 26, avenue Kléber**
**F-75116 Paris(FR)**

**EP 0 246 149 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne de nouveaux agents immunostimulants dérivés du composé dénommé D.25.

Le produit dénommé D.25 est le polysaccharide extrait des protéoglycanes membranaires bactériens composé essentiellement d'unité galactose et ayant un poids moléculaire de 30± 10 kD. Ce polysaccharide a été décrit dans le brevet français n° 84 13844, bien que le poids moléculaire soit présenté comme supérieur.

Ce polysaccharide possède des propriétés immunostimulantes, notamment vis-à-vis de l'induction d'interféron endogène et de l'activation des cellules NK (Natural Killers). Ce polysaccharide est préférentiellement isolé d'une souche mutante non capsulée et non pathogène de Klebsiella pneumoniae biotype a, déposée à la Collection Nationale de l'Institut Pasteur sous le n° 145-I-IP.

La présente invention concerne des dérivés d'hémisynthèse de ce composé D.25, plus particulièrement il s'agit de composés de type amide, ester ou éther du D.25 ainsi que les sels et dérivés d'ammonium quaternaire.

Parmi ces composés, il faut citer les dérivés du D.25 avec des acides, des amines, des amides ou des alcools à chaîne grasse, c'est-à-dire présentant au moins 4 atomes de carbone alicyclique.

Ce type de composés à chaîne lipophile modifie le caractère hydrophile du D.25 et donc son affinité et les interactions avec les membranes cellulaires.

Le greffage sur cette molécule d'une partie hydrophobe permet d'augmenter et/ou de moduler sa capacité d'interaction avec la membrane des cellules immunocompétentes et donc ses propriétés immunostimulantes et adjuvantes.

Ainsi, dans le cas des allergènes, comme par exemple ceux du pollen, du venin des hyménoptères, des acariens, ou l'ovalbumine, qui pourront être avantageusement couplés au D.25, les propriétés immunitaires des conjugués obtenus sont différentes de celles des allergènes initiaux. On peut ainsi orienter la réponse immunitaire vers une protection avec apparition d'IgG au lieu de la réponse allergique associée à l'apparition d'IgE.

Bien entendu, les chaînes peuvent comporter d'autres fonctions ou hétéroatomes selon la nature du composé désiré.

D'autre part, parmi les dérivés du D.25, il faut citer les conjugués du D.25 avec une drogue ayant une activité pharmacologique; dans ce cas, la drogue est fixée au D.25 par l'intermédiaire d'un bras bifonctionnel comportant une fonction acide, amine ou alcool, comme indiqué précédemment, et une autre fonction dépendant de la drogue à conjuguer.

Parmi les composés en cause, ou peut citer les dérivés de formules :

[D.25]-CO-NH-R (I)

$$[D.25]-NH_2-\underset{\underset{O}{\|}}{C}-R \qquad (II)$$

[D.25]-O-R (III)

dans lesquelles R est un radical aliphatique ou acyl pour (I) et (III) et amino pour (II) qui peut être substitué.

Parmi les radicaux aliphatiques, il faut citer les radicaux alcoyles droits ou ramifiés, ayant de préférence de 1 à 30 atomes de carbone, de préférence de 4 à 16 atomes de carbone, ainsi que les radicaux alcényles et alcynyles ayant de 2 à 30 atomes de carbone et de préférence de 4 à 16 atomes de carbone.

Parmi les radicaux acyles, on préfère les radicaux correspondant aux radicaux aliphatiques précédents.

Parmi les radicaux amino, il faut citer les radicaux amino correspondant également aux radicaux aliphatiques précédents.

Les radicaux acyles ou amino peuvent également correspondre à des peptides ou des protéines naturelles ou synthétiques.

Parmi les substituants de R, on peut citer les radicaux amino primaire, secondaire ou tertiaire, les radicaux carboxyles, acyloxy et les radicaux aliphatique-O-et hydroxy.

Les radicaux substituants de R sont, de préférence, des radicaux amino secondaire ou tertiaire, les substituants étant des radicaux aliphatiques qui peuvent être substitués comme défini précédemment par exemple :

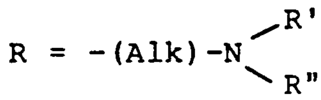

. Alk est un radical alcoylène en $C_4$ à $C_{10}$,

. R′ et R″ sont H, alcoyle en $C_1$ à $C_{16}$ ou hydroxyéthyle,

ainsi que les radicaux acyles correspondants.

Comme précédemment, les substituants de R peuvent être les radicaux acyles ou amino correspondant à des peptides ou des protéines naturelles ou synthétiques.

Parmi les composés en cause, il faut citer ceux dans lesquels R comporte comme substituant une drogue ayant une activité du même type que l'activité du D.25, ou une activité associée, par exemple l'acide rétinoïque.

Par"activité associée", on entend désigner, soit une activité complétant celle du D.25, soit une activité exaltant l'activité du D.25. Il peut, enfin, s'agir de composés destinés à vectoriser le D.25 ou bien, au contraire, à être vectorisés par le D.25.

Dans le cas où R comporte un peptide ou une protéine, il s'agit de préférence d'un peptide ou d'une protéine présentant en elle-même une activité antigénique, immunogénique et/ou allergénique, par exemple, dont les propriétés sont modulées ou modifées par conjugaison avec le D.25.

Les composés selon la présente invention sont plus particulièrement des immunomodulateurs et des adjuvants destinés à être utilisés comme médicaments dans les applications thérapeutiques ci-après :

. immunostimulants des défenses immunitaires non spécifiques,

. adjuvants pour les vaccins microbiens ou viraux,

. anticancéreux,

. inducteurs d'interféron endogène,

. activateurs du système NK et des lymphocytes cytotoxiques.

. "carrier" sur lequel peuvent être couplés des allergènes tels que ovalbumine, allergènes d'accariens, d'hyménoptères, de pollen ...

Les composés selon l'invention peuvent être obtenus par des procédés connus.

En particulier, il est possible de coupler le [D.25] et la fraction dérivée tel que R-X par les procédés de couplage mis en oeuvre dans la synthèse des peptides que X soit une fonction amine ou acide.

Ainsi, on peut faire réagir le composé D.25 avec le composé R-X en présence d'un agent de couplage tel qu'une carbodiimide, après avoir si nécessaire activé une ou plusieurs fonctions et protégé d'autres fonctions, comme cela est connu dans ce type de synthèse.

Il est possible également de mettre en oeuvre les techniques de couplage connues, telles que le couplage avec le glutaraldéhyde.

Pour obtenir des éthers, il peut être nécessaire d'activer les groupes hydroxyles ; il s'agit là d'une technique connue dans la synthèse des sucres.

L'invention concerne également l'utilisation de ces nouveaux dérivés à titre d'agents immunostimulants, immunomodulateurs et adjuvants.

En particulier, l'invention concerne des compositions pharmaceutiques comportant au moins un tel agent, et, de préférence, un composé immunogénique ou un produit cytotoxique.

Les exemples ci-après sont destinés à mettre en évidence d'autres caractéristiques et avantages de la présente invention.

EXEMPLE 1

PROCEDE DE FABRICATION DU D.25

1) Isolement du protéoglycane membranaire brut

1.1) La biomasse de la souche de Klebsiella pneumoniae 145-I-IP est dispersée dans du tampon Tris-HCl (10 mM) pH 7,0 glacé contenant NaCl (0,15 M), puis soumise à un broyage mécanique destiné à rompre les parois cellulaires.

1.2) Le lysat bactérien est clarifié par centrifugation continue à 15 000 g et le surnageant recueilli.

1.3) Le surnageant est traité par addition d'acide trichloracétique, q.s.p. 5 % (P/V), à froid pour éliminer les impuretés (acides nucléiques et protéines) par précipitation.

1.4) Le précipité est éliminé par centrifugation continue à 15 000 g. Le surnageant limpide est recueilli

puis neutralisé par NaOH.

1.5) La solution est alors dialysée puis concentrée par ultrafiltration continue sur membranes Millipore coupant à 10 000 Daltons.

* La solution concentrée obtenue à ce stade correspond au protéoglycane membrane brut.

2) Isolement de la fraction polysaccharidique brute

2.1) Hydrolyse alcaline ménagée du protéoglycane membranaire : cette opération est destinée à dépolymériser le protéoglycane membranaire brut pour libérer la fraction polysaccharidique, dans les conditions suivantes :

La solution concentrée de protéoglycane membranaire brut obtenue précédemment est additionnée de NaOH concentré pour avoir une concentration finale de 0,5 M en NaOH. On hydrolyse ensuite pendant 1 heure à 56° C. Après refroidissement rapide, la solution est neutralisée par HCl.

2.2) La solution neutralisée est clarifiée par filtration sur filtre-presse puis concentrée par ultrafiltration sur membrane Millipore coupant à 10 000 Daltons.

* La solution concentrée obtenue à ce stade correspond à la fraction polysaccharidique brute.

3) Purification de la fraction polysaccharidique

3.1) Le concentrat obtenu au paragraphe 2.2 est soumis à une première chromatographie sur colonne moulée avec du Sephacryl S 1 000 (Pharmacia) dans le tampon Tris-HCl (10 mM) pH 7, ce qui permet d'éliminer les contaminants de haut poids moléculaire contenus dans le pic d'exclusion.

3.2 Le pic d'élution de la colonne contenant la fraction polysaccharidique est encore faiblement contaminé par des protéines de poids moléculaire très proche (70 000 à 100 000). Ces protéines sont hydrolysées 2 heures à 37° C par action de la protéinase K à 50 µg/ml dans le tampon Tris-HCl (10 mM) pH 7 contenant EDTA (1 mM).

3.3) Les protéines contaminantes dont le poids moléculaire a été réduit par protéolyse sont séparées de la fraction polysaccharidique par chromatographie sur colonne de Sephacryl S 200 (Pharmacia) dans l'eau distillée.

3.4) Le pic d'élution contenant la fraction polysaccharidique purifiée est recueilli, puis concentré par ultrafiltration sur membrane Millipore coupant à 10 000 daltons.

4) Obtention du D.25 à partir de la fraction polysaccharidique

La fraction polysaccharidique obtenue au paragraphe 3.4 renferme quelques molécules d'acides gras qui sont éliminés après hydrolyse acide ménagée pour libérer des sites de couplage en vue de la préparation des dérivés d'hémisynthèse du D.25.

4.1) La solution concentrée obtenue au paragraphe 3.4 est additionnée d'acide acétique concentré à raison de 1 % (V/V) puis chauffée à 90° C pendant 90 minutes.

4.2) Après refroidissement rapide, les substances lipidiques dissociées du D.25 précipitent et sont séparées par centrifugation.

4.3) Le surnageant ainsi obtenu est neutralisé par NaOH dilué puis dialysé et concentré par ultrafiltration sur membrane Millipore coupant à 10 000 daltons.

4.4) La solution concentrée est filtrée sur membrane 0,2 µm puis le filtrat est lyophilisé.

* Ce lyophilisat correspond au D.25 faisant l'object de la présente invention et à partir duquel seront réalisés les dérivés d'hémisynthèse. Son poids moléculaire est alors voisin de 30 ± 10 kD.

EXEMPLE 2

STRUCTURE DU D.25

La structure du D.25 comporte, d'une part une chaîne polysaccharidique linéaire constituée par la répétition environ 5 fois d'un motif monomérique de 10 sucres, et, d'autre part, d'une séquence de liaison unique, plus complexe, à laquelle sont liées de courtes chaînes peptidiques.

L'unité monomérique répétitive de la chaîne polysaccharidique linéaire ne renferme que du galactose sous forme pyrane et furane dans les proportions suivantes :

3 $\beta$ Gal p, 3 $\alpha$ Gal p, 2 $\beta$ Gal f, 2 $\alpha$ Gal f.

La séquence de cette unité monomérique est la suivante :

/‾PS_7 = ... —/‾→ β Gal p —→ β Gal f —→ α Gal p —→
1,3 1,3 1,3

β Gal p —→ α Gal f —→ α Gal p —→ α Gal f —→ β Gal p
1,3 1,3 1,3 1,3

—→ α Gal p —→ β Gal f —_7→ structure de liaison
1,3 1,3 1,3

La structure unique de liaison est fixée à l'extrémité de la chaîne polysaccharidique linéaire. Elle contient des résidus glucose, galactose, glucosamine, heptose et acide mannodéoxyoctulosonique. De courtes chaînes peptidiques sont liées à cette structure.

La séquence probable est la suivante :

/‾PS_7—/‾→ α Glc p —→ α Gal p —→ α Hep p —→ α Hep p —→ Man Oc.
1,3 1,2 1,3 1,3 1,5

↑

α Glc p $NH_2$

Les amino-acides suivants composent les chaînes peptidiques associées :

Acide aspartique (3)
Acide glutamique (2)
Sérine (1)
Proline (1)
Glycine (1,5)
Alanine (2)
Valine (1)
Leucine (1)
Lysine (1)

<u>Abréviations</u> :

| | |
|---|---|
| β Gal p = | β Galactopyranose |
| α Gal p = | α Galactopyranose |
| β Gal f = | β Galactofuranose |
| α Gal f = | α Galactofuranose |
| α Glc p = | α Glucopyranose |
| α Glc p, $NH_2$ = | α Glucosamine |
| α Hep p = | α Heptose (D. Mannoheptose) |
| Man Oc. A = | 3-deoxy-D-manno-octulosonic acid |

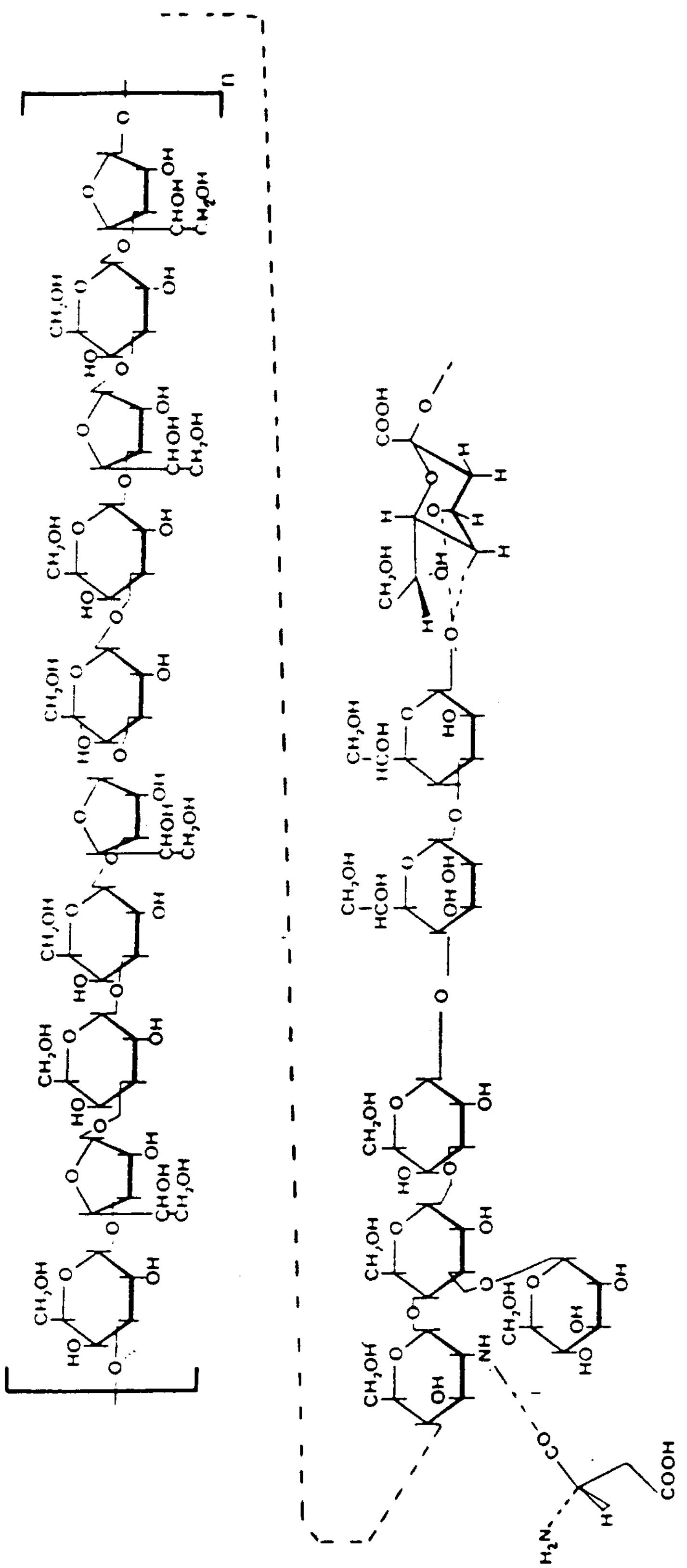

STRUCTURE du D25

EXEMPLE 3

COUPLAGE D N-BUTYRAMIDE AU D.25
(N-butyramide = $CH_3-CH_2-CH_2-CONH_2$ ; PM = 87,12)

a) 100 mg de D.25 sont dissous dans 5 ml d'eau distillée et le pH de la solution est ajusté à pH 4,5 par HCl dilué.

b) 3 mg de N-butyramide sont dissous dans 1 ml d'eau distillée. On ajoute à cette solution 1 ml d'une solution aqueuse à 6 mg/ml d'EDCI (1-éthyle-3-(3-diméthylaminopropyl)-carbodiimide hydrochloride). Le pH est ajusté à pH 4,5 puis on agite pendant 1 heure à température ambiante.

Les deux solutions (a) et (b) sont mélangées puis agitées pendant 1 nuit à température ambiante.

Le complexe D.25-butyramide ainsi formé est ensuite débarrassé des excès de réactifs par chromatographie sur une colonne de Séphadex G.25 (Pharmacia) de 2 x 60 cm dans l'eau distillée.

Le pic d'exclusion contenant le complexe D.25-butyramide est recueilli puis lyophilisé.

EXEMPLE 4

COUPLAGE DE L'OCTYLAMINE AU D.25
(octylamine = $CH_3(CH_2)_6$-$CONH_2$ ; PM = 129,2)

a) 100 mg de D.25 sont dissous dans 5 ml d'eau distillée et le pH de la solution est ajusté à pH 4,5 par HCl dilué.

b) 5 mg d'octylamine sont dissous dans 4 ml d'alcool éthylique puis on ajoute à cette solution 1 ml d'une solution aqueuse contenant 20 mg de CMCI (1-cyclohexyle-3-(2-morpholinoethyl)-carbodiimide). Le pH est ajusté à 4,5 puis on agite pendant 1 heure à température ambiante.

Les deux solutions (a) et (b) sont mélangées puis agitées pendant 1 nuit à température ambiante.

L'excès de réactifs est ensuite séparé du complexe par chromatographie sur une colonne de Séphadex LH-20 (Pharmacia) préalablement équilibrée avec de l'éthanol à 50 %.

Le pic contenant le complexe est recueilli au volume d'exclusion et le complexe est précipité par addition de 4 volumes d'acétone. Le précipité est recueilli par filtration, lavé par l'acétone puis séché sous vide sur $P_2 0_5$.

EXEMPLE 5

COUPLAGE DE L'ACIDE DODECANOIQUE AU D.25
(acide dodécanoïque = $CH_3(CH_2)_{10}$-COOH ; PM = 200,32)

a) 100 mg de D.25 sont dissous dans 5 ml d'eau distillée.

b) 5 mg d'acide dodécanoïque sont dissous dans 5 ml de THF (tétrahydrofuranne) puis on ajoute à cette solution 6 mg de EEDQ (N-éthoxycarbonyl-2-éthoxy-1,2-dihydroquinoline). On agite 1 heure à température ambiante.

Les deux solutions (a) et (b) sont mélangées et agitées une nuit à température ambiante.

L'excès d'acide gras non lié est ensuite éliminé au moyen de plusieurs extractions au chloroforme et le complexe D.25-acide dodécanoïque est dialysé contre l'eau distillée puis lyophilisé.

EXEMPLE 6

COUPLAGE D'UN DIAMINOSPACER AU D.25 POUR PERMETTRE LE COUPLAGE ULTERIEUR DE SUBSTANCES AYANT UN CARBOXYLE LIBRE
(exemple : couplage 1,4-diaminobutane-D.25 = D.25-DAMB)

50 mg de D.25 et 300 mg de diaminobutane dans 50 ml de tampon phosphate de potassium (0,1 M) pH 6,1 sont additionnés de 300 mg de EDC (1-éthyl-3-(3-déméthylaminopropyl)-carbodiimide, HCl). Après deux heures d'agitation à température ambiante, le mélange est dialysé contre du tampon $NaHCO_3$ (0,05 M) pH 8,3 à 4 ° C, changé plusieurs fois pour éliminer les réactifs en excès.

Le dialysat est lyophilisé = D.25-DAMB.

EXEMPLE 7

COUPLAGES D'UNE POLYLYSINE AU D.25-DAMB OBTENU A L'EXEMPLE 7

50 mg de D.25-DAMB et 60 mg de polylysine de PM 5 000 - 10 000 dans 5 ml d'eau distillée sont additionnés de 20 mg de cyanamide (Sigma). Après agitation 1 nuit à température ambiante le mélange est

dialysé contre de l'eau distillée et le pic contenant le complexe (1 pic d'élution) est recueilli puis lyophilisé : D.25-polylysine.

EXEMPLE 8

REALISATION D'UN DERIVE D.25-DAMB-ACIDE RETINOIQUE

25 mg de D.25-DAMB et 5 mg d'acide rétinoïque sont dissous dans 3 ml de tétrahydrofurane. On ajoute à cette solution 18 mg de DCCI (dicyclohexyl-carbodiimide) puis on laisse en agitation pendant 18 heures à température ambiante.

On ajoute ensuite 3 ml d'eau distillée puis on procède à une série d'extraction par le chloroforme pour extraire l'excès de réactifs. Le dérivé D.25-acide rétinoïque présent dans la phase aqueuse est dialysé contre de l'eau distillée puis lyophilisé.

EXEMPLE 9

COUPLAGE DE L'OVALBUMINE (ova) AU D.25

100 mg de D.25 et 100 mg d'ova sont dissous dans 10 ml d'eau distillée. Le pH de la solution est amené à 4,7 avec HCl 0,1 N. On ajoute à cette solution 1 ml d'une solution contenant 0,5 mg de CMCI (1-cyclohexyl-3(2-morpholinoéthyl)-carbodiimide). On agite alors pendant 1 heure à température ambiante en maintenant le pH à 4,7 puis une nuit à + 10° C.

Le conjugué D.25-ova est purifié par chromatographie sur colonne de Sephacryl S-200 (Pharmacia) en tampon phosphate 0,1 M pH 7,4. Le pic contenant le conjugué D.25-ova (premier pic d'élution) est recueilli, dialysé, puis lyophilisé.

Le conjugué ainsi obtenu contient 1 molécule d'ovalbumine par molécule de D.25.

**Revendications**

**Revendications pour les Etats contractants suivants : AT, BE, CH, DE, FR, GB, IT, LI, LU, NL, SE**

**1.** Dérivé polysaccharidique caractérisé en ce qu'il s'agit d'un amide, d'un ester, d'un éther, d'un sel ou d'un dérivé d'ammonium quaternaire du D.25 avec une amine, un amide, un acide ou un alcool.

**2.** Dérivé selon la revendication 1, caractérisé en ce que le dérivé est obtenu avec une amine, un acide ou un alcool gras.

**3.** Dérivé selon la revendication 1, caractérisé en ce que l'amine, l'acide ou l'alcool contient au moins 4 atomes de carbone alicyclique.

**4.** Dérivé selon la revendication 1, caractérisé en ce que l'amine, l'acide ou l'alcool est couplé avec une drogue ayant une activité pharmacologique.

**5.** Dérivé selon la revendication 1, caractérisé en ce qu'il présente la formule :

[D.25]-CONH-R (I)
[D.25]-NH-CO-R (II)
[D.25]-O-R (III)

dans lesquelles R est un radical aliphatique ou acyl pour (I) et (III) et amino pour (II) qui peuvent être substitués.

**6.** Dérivé selon la revendication 5, caractérisé en ce que les substituants eventuels sont choisi parmi les radicaux amino primaire, secondaire ou tertiaire, les radicaux carboxy, acyloxy ou alcoxycarbonyl, les radicaux hydroxy et aliphatique-oxy.

**7.** Dérivé selon l'une des revendications 5 et 6, caractérisé en ce que le radical R comporte de 1 à 30 atomes de carbone alicyclique.

8. Dérivé selon la revendication 5, caractérisé en ce que le radical R comporte de 4 à 16 atomes de carbone alicyclique.

9. Dérivé selon la revendication 5, caractérisé en ce que R est le radical d'un peptide ou d'une protéine.

10. Dérivé selon la revendication 5, caractérisé en ce que le substituant de R est le radical d'un peptide ou d'une protéine.

11. Dérivé selon la revendication 5, caractérisé en ce que :

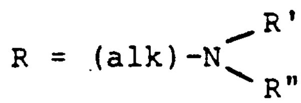

alk est un radical alcoylène en $C_4$ à $C_{10}$, R′ et R" sont, indépendamment, H, alcoyle en $C_1$ à $C_{10}$ ou hydroxyéthyle, ou bien une drogue.

12. Dérivé selon la revendication 5, caractérisé en ce que :

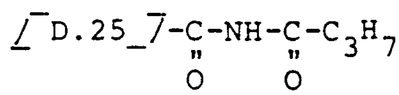

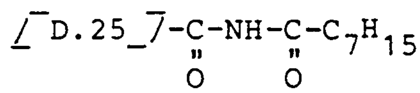

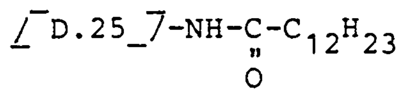

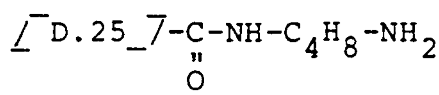

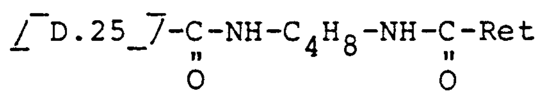

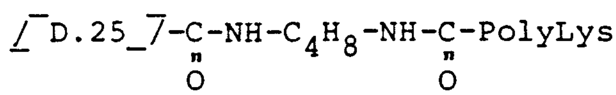

[D.25]-allergène

Ret étant le radical de l'acide rétinoïque,
PolyLys étant le radical de la polylysine.

13. Agent immunostimulant selon l'une des revendications 1 à 12.

14. Composition thérapeutique comportant au moins un agent selon la revendication 13.

15. Composition selon la revendication 14, comportant en outre un composé immunogénique ou un produit cytotoxique.

**Revendications pour les Etats contractants suivants : ES, GR**

1. Procédé de préparation d'un dérivé polysaccharidique caractérisé en ce qu'il s'agit d'un amide, d'un ester, d'un éther, d'un sel ou d'un dérivé d'ammonium quaternaire du D.25 par couplage du D.25 avec une amine, un amide, un acide ou un alcool.

**2.** Procédé selon la revendication 1, caractérisé en ce que le dérivé est obtenu avec une amine, un acide ou un alcool gras.

**3.** Procédé selon la revendiction 1, caractérisé en ce que l'amine, l'acide ou l'alcool contient au moins 4 atomes de carbone alicyclique.

**4.** Procédé selon la revendication 1, caractérisé en ce que l'amine, l'acide ou l'alcool est couplé avec une drogue ayant une activité pharmacologique.

**5.** Procédé selon la revendication 1, caractérisé en ce que le dérivé présente la formule :

[D.25]-CONH-R (I)
[D.25]-NH-CO-R (II)
[D.25]-O-R (III)

dans lesquelles R est un radical aliphatique ou acyl pour (I) et (III) et amino pour (II) qui peuvent être substitués.

**6.** Procédé selon la revendication 5, caractérisé en ce que les substituants éventuels sont choisis parmi les radicaux amino primaire, secondaire ou tertiaire, les radicaux carboxy, acyloxy ou alcoxycarbonyl, les radicaux hydroxy et aliphatique-oxy.

**7.** Procédé selon l'une des revendications 5 et 6, caractérisé en ce que le radical R comporte de 1 à 30 atomes de carbone alicyclique.

**8.** Procédé selon la revendication 5, caractérisé en ce que le radical R comporte de 4 à 16 atomes de carbone alicyclique.

**9.** Procédé selon la revendication 5, caractérisé en ce que R est le radical d'un peptide ou d'une protéine.

**10.** Procédé selon la revendication 5, caractérisé en ce que le substituant de R est le radical d'un peptide ou d'une protéine.

**11.** Procédé selon la revendication 5, caractérisé en ce que :

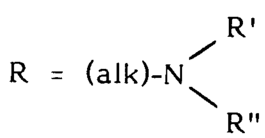

alk est un radical alcoylène en $C_4$ à $C_{10}$, R' et R" sont, indépendamment, H, alcoyle en $C_1$ à $C_{10}$ ou hydroxyéthyle, ou bien une drogue.

**12.** Procédé selon la revendication 5, caractérisé en ce que

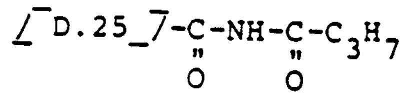

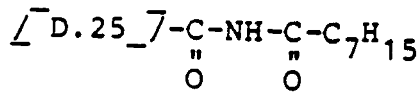

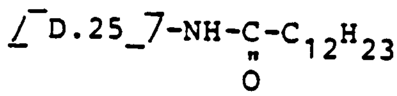

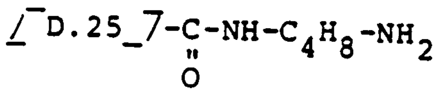

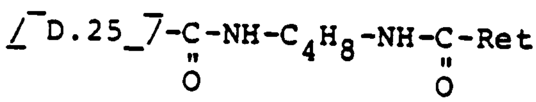

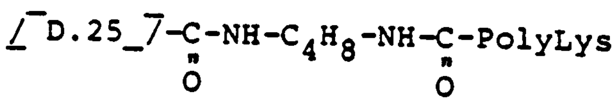

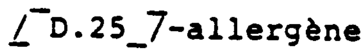

Ret étant le radical de l'acide rétinoïque,
PolyLys étant le radical de la polylysine.

## Claims

## Claims for the following Contracting States : AT, BE, CH, DE, FR, GB, IT, LI, LU, NL, SE

1. A polysaccharide derivative which is an amide, an ester, an ether, a salt or a quaternary ammonium derivative of D.25 with an amine, an amide, an acid or an alcohol.

2. The derivative as claimed in claim 1, wherein the derivative is obtained with a fatty amine, acid or alcohol.

3. The derivative as claimed in claim 1, wherein the amine, acid or alcohol contains at least 4 alicyclic carbon atoms.

4. The derivative as claimed in claim 1, wherein the amine, acid or alcohol is coupled with a drug having a pharmacological activity.

5. The derivative as claimed in claim 1, which possesses the formula:

   [D.25]-CONH-R (I)
   [D.25]-NH-CO-R (II)
   [D.25]-O-R (III)

   in which R is an aliphatic or acyl radical for (1) and (III) and an amino radical for (II), which radicals can be substituted.

6. The derivative as claimed in claim 5, wherein the possible substituents are chosen from primary, secondary or tertiary amino radicals, carboxyl, acyloxy or alkoxycarbonyl radicals and hydroxyl and aliphatic-oxy radicals.

7. The derivative as claimed in one of claims 5 and 6, wherein the radical R contains from 1 to 30 alicyclic carbon atoms.

8. The derivative as claimed in claim 5, wherein the radical R contains from 4 to 16 alicyclic carbon atoms.

**9.** The derivative as claimed in claim 5, wherein R is the radical derived from a peptide or a protein.

**10.** The derivative as claimed in claim 5, wherein the substituent of R is the radical derived from a peptide or a protein.

**11.** The derivative as claimed in claim 5, wherein:

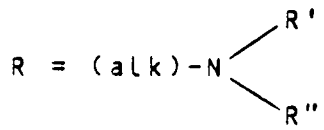

alk is a $C_4$ to $C_{10}$ alkylene radical, and R' and R" are, independently, H, $C_1$ to $C_{10}$ alkyl or hydroxyethyl, or alternatively a drug.

**12.** The derivative as claimed in claim 5, which possesses the formula:

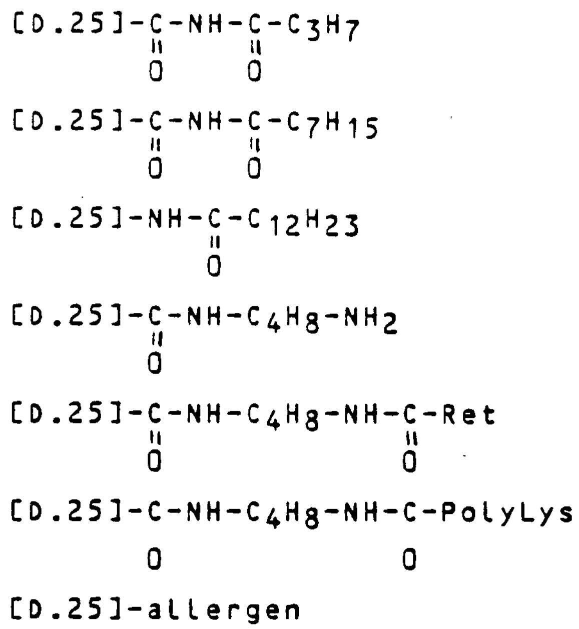

Ret being a retinoic acid radical,
PolyLys being a polylysine radical.

**13.** An immunostimulatory agent as claimed in one of claims 1 to 12.

**14.** A therapeutic composition containing at least one agent as claimed in claim 13.

**15.** The composition as claimed in claim 14, containing in addition an immunogenic compound or a cytotoxic product.

**Claims for the following Contracting States : ES, GR**

**1.** Process for preparing a polysaccharide derivative which is an amide, an ester, an ether, a salt or a quaternary ammonium derivative of D.25 by coupling of D.25 with an amine, an amide, an acid or an alcohol.

**2.** The process as claimed in claim 1, wherein the derivative is obtained with a fatty amine, acid or alcohol.

3. The process as claimed in claim 1, wherein the amine, acid or alcohol contains at least 4 alicyclic carbon atoms.

4. The process as claimed in claim 1, wherein the amine, acid or alcohol is coupled with a drug having a pharmacological activity.

5. The process as claimed in claim 1, which possesses the formula :

[D.25]-CONH-R (I)
[D.25]-NH-CO-R (II)
[D.25]-O-R (III)

in which R is an aliphatic or acyl radical for (I) and (III) and an amino radical for (II), which radicals can be substituted.

6. The process as claimed in claim 5, wherein the possible substituents are chosen from primary, secondary or tertiary amino radicals, carboxyl, acyloxy or alkoxycarbonyl radicals and hydroxyl and aliphaticoxy radicals.

7. The process as claimed in one of claims 5 and 6, wherein the radical R contains from 1 to 30 alicyclic carbons atoms.

8. The process as claimed in claim 5, wherein the radical R contains from 4 to 16 alicyclic carbon atoms.

9. The process as claimed in claim 5, wherein R is the radical derived from a peptide or a protein.

10. The process as claimed in claim 5, wherein the substituent of R is the radical derived from a peptide or a protein.

11. The process as claimed in claim 5, wherein:

R = 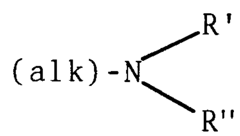

alk is a $C_4$ to $C_{10}$ alkylene radical, and R' and R" are, independently, H, $C_1$ to $C_{10}$ alkyl or hydroxyethyl, or alternatively a drug.

12. The process as claimed in claim 5, which possesses the formula :

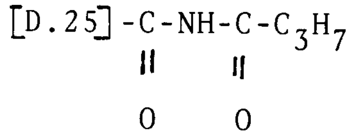

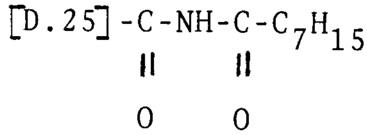

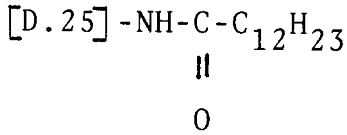

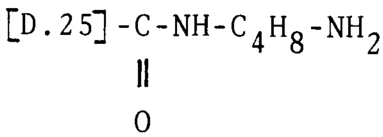

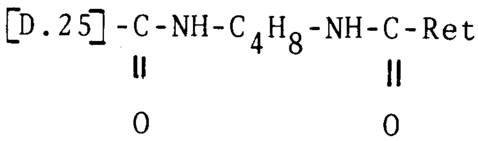

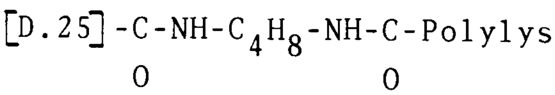

[D.25]-allergen

Ret being a retinoic acid radical,
Polylys being a polylysine radical.

**Patentansprüche**
**Patentansprüche für folgende Vertragsstaaten : AT, BE, CH, DE, FR, GB, IT, LI, LU, NL, SE**

1. Polysaccharidderivat, dadurch gekennzeichnet, daß es sich dabei handelt um ein Amid-, Ester-, Äther-, Salz- oder quaternäres Ammoniumderivat von D.25 mit einem Amin, einem Amid, einer Säure oder einem Alkohol.

2. Derivat nach Anspruch 1, dadurch gekennzeichnet, daß es mit einem Amin, einer Säure oder einem Fettalkohol erhalten wurde.

3. Derivat nach Anspruch 1, dadurch gekennzeichnet, daß das Amin, die Säure oder der Alkohol mindestens 4 alicyclische Kohlenstoffatome enthält.

4. Derivat nach 1, dadurch gekennzeichnet, daß das Amin, die Säure oder der Alkohol mit einem Arzneimittel mit einer pharmakologischen Aktivität gekoppelt ist.

5. Derivat nach Anspruch 1, dadurch gekennzeichnet, daß es die Formel hat:

   [D.25]-CONH-R (I)
   [D.25]-NH-CO-R (II)
   [D.25]-O-R (III)

   in denen R einen aliphatischen Rest oder einen Acylrest für (I) und (III) und einen Aminorest für (II) darstellt, die substituiert sein können.

6. Derivat nach Anspruch 5, dadurch gekennzeichnet, daß die gegebenenfalls vorhandenen Substituenen

ausgewählt werden aus primären, sekundären oder tertiären Aminoresten, Carboxy-, Acyloxy- oder Alkoxycarbonylresten, Hydroxy- und aliphatischen Oxyresten.

**7.** Derivat nach einem der Ansprüche 5 und 6, dadurch gekennzeichnet, daß der Rest R 1 bis 30 alicyclische Kohlenstoffatome enthält.

**8.** Derivat nach Anspruch 5, dadurch gekennzeichnet, daß der Rest R 4 bis 16 alicyclische Kohlenstoffatome enthält.

**9.** Derivat nach Anspruch 5, dadurch gekennzeichnet, daß R der Rest eines Peptids oder eines Proteins ist.

**10.** Derivat nach Anspruch 5, dadurch gekennzeichnet, daß der Substituent von R der Rest eines Peptids oder eines Proteins ist.

**11.** Derivat nach Anspruch 5, dadurch gekennzeichnet, daß R steht für

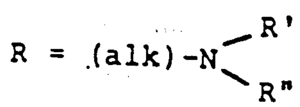

worin Alk einen $C_4$-$C_{10}$-Alkylenrest, R' und R" unabhängig voneinander H, $C_1$-$C_{10}$-Alkyl oder Hydroxyethyl darstellen, oder ein Arzneimittel.

**12.** Derivat nach Anspruch 5, dadurch gekennzeichnet, daß es die Formel hat

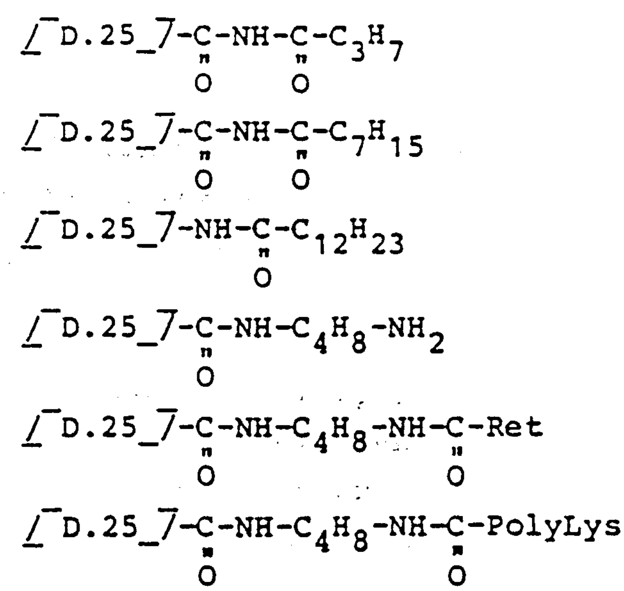

[D.25]- Allergen

worin Ret den Rest der Retinoesäure und PolyLys den Rest von Polylysin darstellen.

**13.** Immunstimulierungsmittel nach einem der Ansprüche 1 bis 12.

**14.** Therapeutische Zusammensetzung, die mindestens ein Agens nach Anspruch 13 enthält.

**15.** Zusammensetzung nach Anspruch 14, die außerdem eine immunogene Verbindung oder ein zytotoxisches Produkt enthält.

**Patentansprüche für folgende Vertragsstaaten : ES, GR**

1. Verfahren zur Herstellung eines Polysaccharidderivats, dadurch gekennzeichnet, daß es sich dabei handelt um ein Amid-, Ester-, Äther-, Salz- oder quaternäres Ammoniumderivat von D.25 durch Kuppeln von D.25 mit einem Amin, einem Amid, einer Säure oder einem Alkohol.

2. Verfahren nach Anspruch 1, dadurch gekennzeichnet, daß das Derivat mit einem Amin, einer Säure oder einem Fettalkohol erhalten wird.

3. Verfahren nach Anspruch 1, dadurch gekennzeichnet, daß das Amin, die Säure oder der Alkohol mindestens 4 alicyclische Kohlenstoffatome enthält.

4. Verfahren nach Anspruch 1, dadurch gekennzeichnet, daß das Amin, die Säure oder der Alkohol mit einem Arzneimittel mit einer pharmakologischen Aktivität gekoppelt wird.

5. Verfahren nach Anspruch 1, dadurch gekennzeichnet, daß das Derivat die Formel hat:

   [D.25]-CONH-R (I)
   [D.25]-NH-CO-R (II)
   [D.25]-O-R (III)

   in denen R einen aliphatischen Rest oder einen Acylrest für (I) und (III) und einen Aminorest für (II) darstellt, die substituiert sein können.

6. Verfahren nach Anspruch 5, dadurch gekennzeichnet, daß die gegebenenfalls vorhandenen Substituenen ausgewählt werden aus primären, sekundären und tertiären Aminoresten, Carboxy-, Acyloxy- oder Alkoxycarbonylresten, Hydroxy- und aliphatischen Oxyresten.

7. Verfahren nach einem der Ansprüche 5 und 6, dadurch gekennzeichnet, daß der Rest R 1 bis 30 alicyclische Kohlenstoffatome enthält.

8. Verfahren nach Anspruch 5, dadurch gekennzeichnet, daß der Rest R 4 bis 16 alicyclische Kohlenstoffatome enthält.

9. Verfahren nach Anspruch 5, dadurch gekennzeichnet, daß R der Rest eines Peptids oder eines Proteins ist.

10. Verfahren nach Anspruch 5, dadurch gekennzeichnet, daß der Substituent von R der Rest eines Peptids oder eines Proteins ist.

11. Verfahren nach Anspruch 5, dadurch gekennzeichnet, daß R steht für

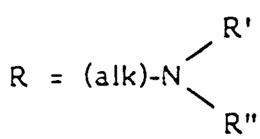

    worin Alk einen $C_4$-$C_{10}$-Alkylenrest, R' und R" unabhängig voneinander H, $C_1$-$C_{10}$-Alkyl oder Hydroxyethyl darstellen, oder auch ein Arzneimittel.

12. Verfahren nach Anspruch 5, gekennzeichnet durch die Formel

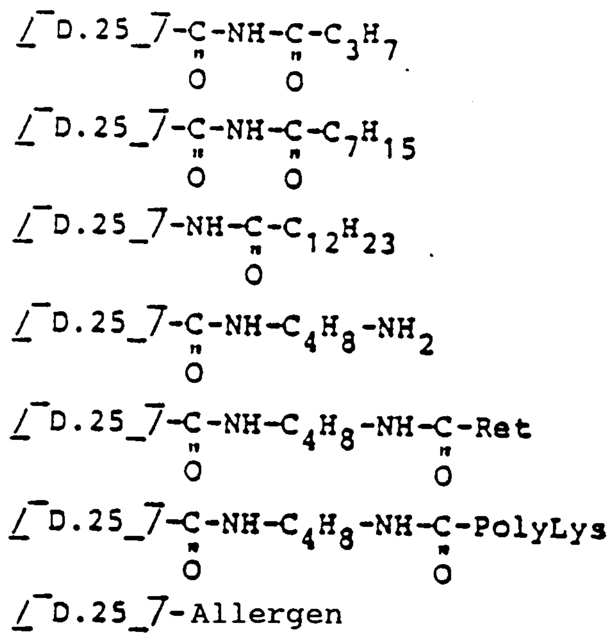

worin Ret den Rest der Retinoesäure und PolyLys den Rest von Polylysin darstellen.